(19) Europäisches Patentamt
European Patent Office
Office européen des brevets

(11) **EP 4 065 039 B1**

(12) **EUROPEAN PATENT SPECIFICATION**

(45) Date of publication and mention
of the grant of the patent:
**15.05.2024 Bulletin 2024/20**

(21) Application number: **20825342.7**

(22) Date of filing: **23.11.2020**

(51) International Patent Classification (IPC):
***A61C 19/06*** (2006.01)

(52) Cooperative Patent Classification (CPC):
**A61C 19/063; A61C 19/066**

(86) International application number:
**PCT/US2020/061717**

(87) International publication number:
**WO 2021/108283 (03.06.2021 Gazette 2021/22)**

(54) **DOSING INDICATOR**

DOSIERVORRICHTUNGSINDIKATOR

INDICATEUR DE DOSAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.11.2019 US 201962940933 P**

(43) Date of publication of application:
**05.10.2022 Bulletin 2022/40**

(73) Proprietor: **The Procter & Gamble Company
Cincinnati, OH 45202 (US)**

(72) Inventor: **WANG, Samantha, Chen-Yee
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
WO-A1-2005/037127      WO-A1-2014/122511
WO-A1-2016/067123      DE-A1-102008 016 987
US-A1- 2014 011 163

**Description**

FIELD OF THE INVENTION

The present invention relates to a dispensing device, such as a whitening pen comprising a dosing indicator for indicating the amount of a whitening composition supplied from the whitening pen.

BACKGROUND OF THE INVENTION

A variety of devices can be used to deliver whitening compositions directly to the teeth. Many whitening compositions, such as those composition comprising peroxide compounds, can lead to sensitivity amongst users. Unfortunately, many users of whitening compositions overdose whitening compositions, which can increase the sensitivity experienced.

Some dosing indicators for pharmaceutical compositions exist but are cost-prohibitive for use in consumer products, such as with whitening compositions. WO2016/7067123A discloses a toothbrush comprising a reservoir that dispenses the compound from the reservoir to the bristles of the head. WO2005/037127A1 discloses a bristle-free peroxide dispensing device and US2014/0011163A1 discloses a vibratory fluid dispensing instrument. However, none of the prior art devices securely and easily inhibits over-dosing. Accordingly, there is a need for an improved cost-effective dosing indicator for a device to deliver whitening compositions.

SUMMARY OF THE INVENTION

Disclosed herein is a cost-effective dosing indicator for devices that dispense consumer products, such as whitening compositions and/or oral care compositions. The dosing indicator comprises only a first and a second dosing indicator symbol, which can convert the distance rotated into amount of material dispensed.

Disclosed herein is a dispensing device 10 as claimed in claim 1.

Preferred embodiments of the dispensing device 10 are disclosed in the dependent claims 2 to 13.

BRIEF DESCRIPTION OF THE DRAWINGS

FIG. 1 is a top view of a device including first and second dosing indicator symbols.
FIG. 2 is a cross-sectional view along a longitudinal axis of the device of FIG. 1.
FIG. 3 is a top view of a device that has been rotated by a user.
FIG. 4 is a cross-sectional view along a longitudinal axis of the device of FIG. 3.

DETAILED DESCRIPTION OF THE INVENTION

The present invention is directed to a dosing mechanism to aid a user to dispense the correct dose of an oral care composition stored within a device. While dosing indicators have been disclosed for devices to dispense pharmaceutical compositions or for devices to be dispensed by a dental professional, there is a need for a cost-effective dosing indicator for use on devices to apply oral care compositions, such as whitening compositions.

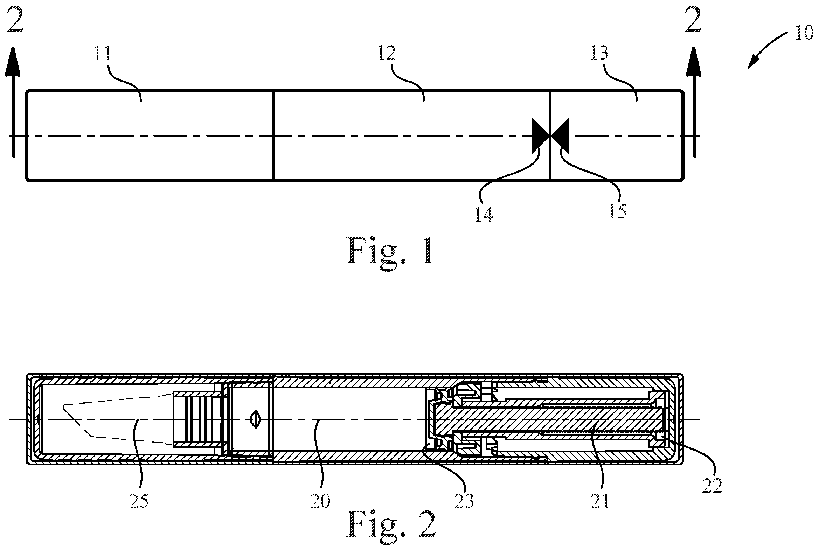

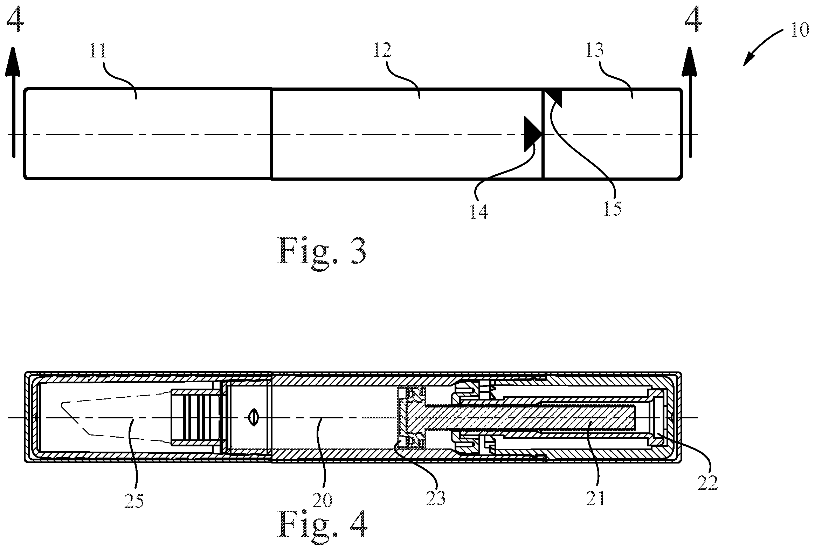

In twisting applicators, as exemplified in the pen-type twist device shown in FIG. 1 and FIG. 3, a user will manually rotate the rotating portion of the device. A rotation of the rotating portion of the device will move a platform at the bottom of a reservoir storing the oral care composition within the device towards an applicator tip, as shown in FIG. 3. The platform lowers the volume in the reservoir, which forces the oral care composition stored within the reservoir, out of one or more orifices in an applicator tip. In current twisting applicators, a user will rotate the rotating portion until a portion of the oral care composition has been expelled onto the applicator tip that can be applied to the oral cavity. Unfortunately, it is difficult to know how much material has been dispensed on the applicator tip beyond visual estimation.

The present invention is directed at a dosing indicator that can guide a user to dispense an exact amount of material. An exact dosing can be beneficial when dispensing oral care compositions comprising a whitening agent, fluoride, or other benefit agents that can require exact dosing to provide the benefit without leading to irritation of the oral cavity and/or extraneous dosing which could lead to accidental ingestion. The rotating portion and body (or stationary portion) include only a first and second dosing indicator symbol so that when the rotating portion is rotated, the user has a guide to how far in distance the rotating portion has been actually rotated. The amount rotated corresponds with the amount of oral care composition extruded onto the applicator tip. A full turn is realized when the first and second dosing indicator symbols start aligned and the rotating portion is rotated a full turn to when the first and second dosing indicator symbols are aligned again, thereby indicating to a user to stop rotating the rotating portion. A full turn of the device corresponds to the correct dosage to apply to the oral care cavity.

Definitions

By "oral care composition", as used herein, is meant a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, tooth gel, subgingival gel, mouth rinse, mousse, foam, tooth whitening composition, tooth coloring composition, or denture care or adhesive product.

"Elastomer," as used herein, is defined as a polymer with rubber-like elasticity. An elastomer is a polymer with viscoelasticity, weak intermolecular forces, low Young's modulus, and high failure strain compared with other polymers and materials.

The term "about" means that amounts, sizes, formulations, parameters, and other quantities and characteristics are not and need not be exact, but can be approximate and/or larger or smaller, as desired, reflecting tolerances, conversion factors, rounding off, measurement errors, and the like, and other factors known to those of skill in the art. In general, an amount, size, formulation, parameter or other quantity or characteristic is "about" or "approximate" whether or not expressly stated to be such. The term "about" also encompasses amounts that differ due to different equilibrium conditions for a composition resulting from a particular initial mixture. Whether or not modified by the term "about," the claims include equivalents to the quantities. The term "about" can mean within 10% of the reported numerical value, preferably within 5% of the reported numerical value.

Several types of ranges are disclosed in the present invention. When a range of any type is disclosed or claimed, the intent is to disclose or claim individually each possible number that such a range could reasonably encompass, including end points of the range as well as any sub-ranges and combinations of sub-ranges encompassed therein.

The foregoing summary is not intended to define every aspect of the invention, and additional aspects are described in other sections. With respect to aspects of the invention described or claimed with "a" or "an," it should be understood that these terms mean "one or more" unless context unambiguously requires a more restricted meaning. The term "or" should be understood to encompass items in the alternative . If aspects of the invention are described as "comprising" a feature, embodiments also are contemplated "consisting of" or "consisting essentially of" the feature.

Features of the compositions and methods are described below. Section headings are for convenience of reading and not intended to be limiting per se. The entire document is intended to be related as a unified disclosure.

Device

The device of the present invention can be shown by FIG. 1. FIG. 1 shows an applicator device (10) comprising a cap (11), a body (12), and a rotating portion (13). The body (12) also comprises a first indicator symbol (14). The rotating portion (13) also comprises a second indicator symbol.

FIG. 2 shows a cross-sectional view taken along a longitudinal axis of the device (10). The device can also comprise a reservoir (20), a rotating member (21), a rotating dial (22), and a platform (23) forming a bottom portion of the reservoir (20). The device can also comprise an applicator (25).

The device (10) can be substantially cylindrical, rectangular, or any other shape suitable for application of an oral care composition to a surface of the oral cavity.

The cap (11) can be a screw cap, an over cap, or any other suitable cap for enclosing the applicator (25) when not in use. The cap (11) can be modified to allow the device (10) to rest on the cap (11) in a vertical position on a suitable surface, such as a countertop.

The body (12) can comprise the reservoir (20), rotating member (21), rotating dial (22), and the platform (23) forming the bottom portion of the reservoir (20). The body (12) can remain stationary when a user rotates the rotating portion (13).

The body (12) can be sized for use in self-application or sized for application by dental professional. The body (12) can be straight or substantially straight. The body (12) can also be curved, angled, or bendable to facilitate application to hard to reach oral care surfaces, such as tooth surfaces in the back of the oral cavity.

The body (12) can be made from any suitable material, such as for example, a polymer, a polymer alloy, an elastomer, a metal, a metal alloy, glass, and/or combinations thereof. The body (12) can have gripping elements or aesthetic elements. The body (12) can be opaque, translucent, transparent, and/or combinations thereof. As described herein, the applicator (25) can be designed to facilitate transfer of an oral care composition from the applicator (25) to an oral cavity surface.

The body (12) can also comprise a ratchet wheel with a top, bottom, and a plurality of teeth at the top of the ratchet wheel. There can also be a securing seat above the ratchet wheel, the securing seat can comprise a top, bottom, and a plurality of teeth opposite to the plurality of teeth of the ratchet wheel.

The applicator (25) can be made from a material that is suitable for application to oral care surfaces. For example, the applicator (25) can be made from food & drug grade materials, materials on the GRAS (Generally Regarded As Safe) list, or other applicable materials approved for use within the oral cavity according to local laws.

The applicator (25) can comprise a tip, one or more bristles, a rollerball, a swab, a needle, or any other suitable applicator for applying a composition to the oral cavity, such as the teeth and/or gums. The applicator tip can be made from any suitable material, such as for example, a polymer, a polymer alloy, an elastomer, a metal, a metal alloy, glass, bristles, and/or combinations thereof. The applicator can be free of bristles.

Suitable polymer materials for the applicator tip include, but are not limited to, nylon, polypropylene, polyethylene, polyethylene terephthalate, and/or combinations thereof.

Suitable elastomer materials include, but are not limited to, thermoplastic elastomers, a styrenic, a copolyester, a polyurethane, a polyamide, a polyolefin blend, a polyolefin alloy, a reactor TPO, a polyolefin plastomer, a polyolefin elastomer, and/or combinations thereof. Suitable elastomers include, for example, an elastomer made from PolyOne® under the Versaflex™ product lines or from Hapco, Inc under the Steralloy™ product line.

The applicator (25) can include one or more orifices to allow the oral care composition stored within the reservoir (20) to be extruded into or out of the applicator (25) to be applied to the oral cavity. The one or more orifices of the applicator (25) can be in fluid communication with the reservoir (20).

The reservoir (20) can be the void created by the inner surface of the body (12), the upper surface of the platform (23), and the applicator (25). The reservoir (20) can comprise the oral care composition until it is dispensed to the applicator (25) and ultimately applied to one or more surfaces within the oral cavity.

The reservoir (20) can comprise from about 0.1 g to about 100 g, from about 0.5 g to about 10 g, from about 1 g to about 5 g, or from about 0.01 g to about 10 g of the oral care composition.

The device (10) can be a twist-up pen-type dispenser, as described in U.S. Patent No. 7,201,527, the apparatus as described in U.S. Patent No. 8,602,774, or the applicator as described in U.S. Patent Application Publication No. 2006/0275225.

Oral Care Composition

The oral care composition that can be dispensed from the reservoir (20) and onto/into the applicator (25) can be any suitable liquid, semiliquid, or any other extrudable composition comprising one or more oral care active agents. For example, the oral care composition can be a whitening composition and include a whitening agent. The oral care composition can include fluoride and/or tin.

The oral care composition may include an effective amount of an anti-caries agent. The oral care composition can comprise a fluoride ion source.

The fluoride ion source may be present in an amount sufficient to give a suitable fluoride ion concentration in the composition according to local laws and regulations, for example the anti-caries monograph at the FDA. The oral care composition can comprise from about 0.0025% to about 20%, from about 0.0025% to about 10%, from about 0.01% to about 5%, or from about 0.0025% to about 2 %, by weight of the oral care composition, of the fluoride ion source.

The fluoride ion source can comprise stannous fluoride, sodium fluoride, potassium fluoride, amine fluoride, sodium monofluorophosphate, zinc fluoride, and/or combinations thereof.

The fluoride ion source and the metal ion source can be the same compound, such as for example, stannous fluoride, which can generate tin ions and fluoride ions. Additionally, the fluoride ion source and the tin ion source can be separate compounds, such as when the metal ion source is stannous chloride and the fluoride ion source is sodium monofluorophosphate or sodium fluoride.

The oral care composition can comprise a metal ion source. Suitable metal ion sources include stannous ion sources, zinc ion sources, copper ion sources, silver ion sources, magnesium ion sources, iron ion sources, sodium ion sources, and manganese (Mn) ion sources, and/or combinations thereof. The metal ion source can be a soluble or a sparingly soluble compound of stannous, zinc, or copper with inorganic or organic counter ions. Examples include the fluoride, chloride, chlorofluoride, acetate, hexafluorozirconate, sulfate, tartrate, gluconate, citrate, malate, glycinate, pyrophosphate, metaphosphate, oxalate, phosphate, carbonate salts and oxides of stannous, zinc, and copper.

Stannous, zinc and copper ions are derived from the metal ion source(s) can be found in the multi-phase oral care composition an effective amount to provide an oral care benefit or other benefits. Stannous, zinc and copper ions have been found to help in the reduction of gingivitis, plaque, sensitivity, and improved breath benefits. An effective amount is defined as from at least about 500 ppm to about 20,000 ppm metal ion of the total composition, preferably from about 2,000 ppm to about 15,000 ppm. More preferably, metal ions are present in an amount from about 3,000 ppm to about 13,000 ppm and even more preferably from about 5,000 ppm to about 10,000 ppm. This is the total amount of metal ions (stannous, zinc, copper and mixtures thereof) that is present in the compositions for delivery to the tooth surface.

Other metal ion sources can include minerals and/or calcium containing compounds, which can lead to remineralization, such as, for example, sodium iodide, potassium iodide, calcium chloride, calcium lactate, calcium phosphate, hydroxyapatite, fluoroapatite, amorphous calcium phosphate, crystalline calcium phosphate, sodium bicarbonate, sodium carbonate, calcium carbonate, oxalic acid, dipotassium oxalate, monosodium monopotassium oxalate, casein phosphopeptides, and/or casein phosphopeptide coated hydroxy apatite.

The metal ion source may comprise a metal salt suitable for generating metal ions in the oral cavity. Suitable metal salts include salts of potassium (K), silver (Ag), magnesium (Mg), iron (Fe), sodium (Na), and manganese (Mn) salts, or combinations thereof. Preferred salts include, without limitation, gluconates, chlorates, citrates, chlorides, fluorides, and nitrates, or combinations thereof.

The oral care composition can comprise at least about 0.005%, from about 0.005% to about 10%, from about 0.01% to about 5%, from about 0.01% to about 2%, or from about 0.1% to about 1% of a metal ion source by weight of the oral care composition.

Tin ions, such as stannous ions, are used in oral care compositions to deliver benefits such as, for example, enamel care and cavity protection. Suitable tin ion sources include stannous chloride, stannous fluoride, stannous bromide, stannous iodide, stannous acetate, stannous gluconate, stannous oxalate, stannous sulfate, stannous lactate, stannous tartrate stannous carbonate, stannic chloride, stannic fluoride, stannic iodide, stannic citrate, stannic nitrate, stannous peptides, stannous proteins, and stannous phosphate, and combinations thereof. Preferably, the ion source is stannous fluoride, stannous chloride, and/or combinations thereof.

The oral care compositions of the present invention may comprise a tin ion source in the amount ranging from about 0.01% to about 5%, from about 0.05% to about 4%, from about 0.01% to about 10%, or from about 0.075% to about 3%.

The oral care composition can also comprise a whitening composition, the whitening composition comprising a whitening agent. The oral care composition may comprise from about 0.1% to about 10%, from about 0.2% to about 5%, from about 1% to about 5%, or from about 1% to about 40%, by weight of the oral care composition, of a whitening agent. The whitening agent can be a compound suitable for whitening at least one tooth in the oral cavity. The whitening agent may include peroxides, metal chlorites, perborates, percarbonates, peroxyacids, persulfates, and combinations thereof. Suitable peroxides include hydrogen peroxide, solid peroxides, urea peroxide, calcium peroxide, benzoyl peroxide, sodium peroxide, barium peroxide, inorganic peroxides, hydroperoxides, organic peroxides, and mixtures thereof. Suitable metal chlorites include calcium chlorite, barium chlorite, magnesium chlorite, lithium chlorite, sodium chlorite, and potassium chlorite. Other suitable whitening agents include sodium persulfate, potassium persulfate, peroxydone, 6-phthalimido peroxy hexanoic acid, Pthalamidoperoxycaproic acid, or mixtures thereof.

The oral care composition can also include those compositions described in in U.S. Patent App. Pub. No. 2018/0133119 and U.S. Patent App. Pub. No. 2018/0133121.

The oral care composition can also include various thickening materials to thicken the composition. The oral care compositions herein may include one or more thickening agents. A thickening agent may be used in an amount from about 0.01% to about 15%, or from about 0.1% to about 10%, or from about 0.1% to about 5%, by weight of the oral care composition. Non-limiting examples may include those described in US 2008/0081023 A1 at paragraphs 134 to 137.

The oral care composition can comprise a linear sulfated polysaccharide as a thickening agent. Carageenans or carageenans are one example of a linear sulfated polysaccharide. Generally, carageenans can vary based upon the degree of sulfation that includes: Kappa-carrageenan, Iota-carrageenan, and Lambda-carrageenan. Combinations of carageenans can be used. The oral care composition can contain from about 0.1% to about 3%, of a linear sulfated polysaccharides by weight of the oral care composition, from about 0.5% to about 2%, from about 0.6% to about 1.8%, or combinations thereof.

The oral care composition can comprise a silica agent, preferably a thickening silica obtained from sodium silicate solution by destabilizing with acid as to yield very fine particles. One commercially available example is ZEODENT® branded silicas from Huber Engineered Materials (e.g., ZEODENT® 103, 124, 113 115, 163, 165, 167). The oral care composition can include from about 0.5% to about 5% by weight of the oral care composition of a silica agent, preferably from about 1% to about 4%, alternatively from about 1.5% to about 3.5%, alternatively from about 2 % to about 3%, alternatively from about 2% to about 5% alternatively from about 1% to 3%, alternatively combinations thereof.

The thickening agent can comprise a carboxymethyl cellulose ("CMC"). CMC is prepared from cellulose by treatment with alkali and monochloro-acetic acid or its sodium salt. Different varieties are commercially characterized by viscosity. One commercially available example is Aqualon™ branded CMC from Ashland Special Ingredients (e.g., Aqualon™ 7H3SF; Aqualon™ 9M3SF Aqualon™ TM9A; Aqualon™ TM12A). The thickening agent can contain from about 0.1% to about 3% of a CMC by weight of the oral care composition, preferably from about 0.5% to about 2%, alternatively from about 0.6% to about 1.8%, alternatively combinations thereof.

Dispensing the Oral Care Composition

The oral care composition can be dispensed out of the device (10) by having a user rotate the rotating portion (13).

Upon rotation of the rotation of the rotating portion (13), the rotating dial (22) can be rotated, which can cause the rotating member (21) to move towards the applicator (25). The rotation of the rotating portion (13) can also cause a ratchet wheel with a top, bottom, and a plurality of teeth at the top to rotate. Each time a tooth of the ratchet wheel rotates, an audible click can be heard, as the plurality of teeth of the ratchet wheel interact with the plurality of teeth of the securing seat.

The rotating member (21) can be threaded with screw threads for mounting to the interior surface of the body (12), as shown in FIG. 2. The platform (23) can be associated with and/or attached to the rotating member (21). When the rotating dial (22) is rotated in one direction, the rotating member (21) can rotate in the same direction within the screw threads, as shown in FIG. 4. The movement of the rotating member (21) can move the platform (23) to squeeze the oral care composition towards the one or more orifices of the applicator (25). This process is well known in the art and further described in detail in U.S. Patent No. 7,201,527.

Indicator Symbols

When a user rotates the rotating portion (13), it can be challenging for the user to quantify the amount of material squeezed from the reservoir (20) and onto/into the applicator (25). Thus, the present invention is directed towards dosing indicator symbols to denote the distance the user has rotated the rotating portion (13).

As shown in FIG. 1, the body (12) comprises a first dosing indicator symbol (14) and the rotating portion (13) includes a second dosing indicator symbol (15). FIG. 1 displays the device when the first (14) and second (15) dosing indicator symbols are aligned. FIG. 3 displays the device when the second dosing indicator symbol (15) has been rotated in one direction with the rotating portion (13) of the device (10). As shown in FIG. 4, the rotation of the rotating portion (13) pushing the platform (23) towards the applicator (25) and expels a portion of the composition from the reservoir (20).

A defined distance of the movement of the second dosing indicator (15) corresponds to an amount of composition dispensed from the device (10). For example, a user can be instructed to turn the rotating portion (13) a full turn, thereby starting and stopping the rotation when the first (14) and second (15) dosing indicator symbols are aligned.

The dosing indicator symbol can be any visual and/or aesthetic feature that can allow the user to determine how far the rotating portion (13) has been rotated. The dosing indicator symbol(s) can be a printed symbol, color, shape, graphic, picture, word, logo, etc. on the packaging sleeve or on the device (10) itself or the dosing indicator symbols can be a raised or depressed feature of the device (10), such as embossing, debossing, ridge, or the like. Suitable examples of dosing indicator symbols can include, but are not limited to, arrows, dots, circles, rectangles, triangles, lines, words, such as "START" and/or "STOP", circles, ovals, letters, figures, or the like. Dosing indicator symbols can complete a graphic when aligned properly. For example, a portion of a graphic can be on the body (12) and the remaining portion of a graphic can be on the rotating portion (13). When aligned, the graphic is visually complete.

The dosing indicator symbol(s) can also differ in transparency from the body (12) and/or rotating portion (13). For example, if the body (12) is transparent or translucent, the dosing indicator symbol(s) can be opaque.

The first (14) and second (15) dosing indicator symbols can be identical, substantially similar, or different in appearance. The first dosing indicator symbol (14) can be a symbol, as described herein, while the second dosing indicator symbol (15) can be a scale based on mass, volume, and/or weight that spans the at least a portion of the outer surface of the rotating portion (13). The first (14) and second (15) dosing indicator symbols can be in direct contact when aligned, can have a gap between the first (14) and second (15) dosing indicator symbols when aligned, and/or the first (14) and second (15) dosing indicator symbols can be substantially visually aligned without direct contact.

A full turn of the rotating portion (13) is when the first (14) and second (15) dosing indicator symbols start aligned and are rotated in one direction until the first (14) and second (15) dosing indicator symbols become aligned again. A full turn of the rotating portion (13) corresponds to the dispensing of from about 0.05 g to about 0.25 g, or about 0.1 g of the oral care composition.

In the example where the oral care composition is a whitening composition and the whitening composition comprises hydrogen peroxide, a full turn of the device (10) can correspond to the correct dosage of hydrogen peroxide that should be applied to the oral cavity in a single application to effectively whiten teeth without experiencing irritation.

In the example where the oral care composition comprises fluoride, a full turn of the device (10) can correspond to the correct dosage fluoride that should be applied to the oral cavity in a single application according to the relevant regulatory agency, such as the United Stated Food and Drug Administration (FDA).

Methods

Also disclosed herein is a non-claimed method for dispensing and/or applying and the medical use of an oral care composition using the dosing indicator symbols and illustrated in FIG. 1-4.

A user can dispense a defined amount of an oral care composition by using the dosing indicator symbols as shown in FIG. 1-4. First, a user can align the first (14) and second (15) dosing indicator symbols as shown in FIG. 1. Next, a user can rotate the rotating portion (13) of the device (10) in one direction as shown in FIG. 3. As described herein, the rotation of the rotating portion (13) dispenses the oral care composition stored within the reservoir (20) from the device (10) and into/onto the applicator (25). The user can stop rotating the rotation portion (13) once the first (14) and second (15) dosing indicator symbols are aligned. Finally, the oral care composition can then be applied to an oral cavity surface, such as the teeth in the example of fluoride or peroxide or the teeth and/or the gums in the case of tin.

EXAMPLES

The invention is further illustrated by the following examples, which are not to be construed in any way as imposing limitations to the scope of this invention.

A whitening pen was provided by WSD Labs and the reservoir of the whitening pen was loaded with the whitening composition of TABLE 1.

TABLE 1. Whitening Composition

| | |
|---|---|
| Hydrogen Peroxide (35%) | 8.700 |
| Glycerin, USP | 20.500 |
| Water | 65.400 |
| Sodium Acid Pyrophosphate | 1.000 |
| Carbopol® 956 Polymer[3] (CAS# is 134499-38-0) | 2.000 |
| Sodium Hydroxide (50% solution) | 0.900 |
| Flavor | 1.000 |
| Sucralose, USP | 0.500 |
| [3]Available from the Goodrich Corporation (Akron, Ohio, USA) | |

It was observed that a full rotation of the rotating portion was 24 "clicks" or 24 movements of the internal ratchet wheel within the body of the whitening pen. Thus, an experiment was conducted to measure the amount of material that was dispensed from the pen after a full rotation of the rotating portion. The whitening pen was weighed. Then, the rotating portion was rotated by a defined number of "clicks" to dispense an amount of the whitening composition. The whitening composition was then removed from the applicator tip in a manner similar to how a user would apply a composition to an oral cavity. The whitening pen was then re-weighed. The displaced weight was equivalent to the amount of material dispensed.

The results of the dosing check are described in TABLE 2. In the first trial, after 25 "clicks," only 0.04 g of material was dispensed onto the oral cavity. This was due to the fact that the headspace above the reservoir of the whitening composition was empty prior to the first use. Thus, less material was dispensed with the first full rotation.

After the initial priming of the whitening pen, subsequent rotations led to 0.1 g, 0.1 g, 0.12 g, and 0.11 g of dispensed material based on the # of clicks provided in TABLE 2. If trial 1 is excluded, approximately 0.1g of material was applied to the oral cavity. Additionally, demarcations were added on the device to calculate the # of clicks that represented a full rotation. This resulted in the observation that in the whitening pen utilized, a full rotation was represented with 24 "clicks." As such, using the averaged dispensed material per full rotation was calculated by Formula I.

TABLE 2: Dispensed Composition

| Trial | Initial weight of Pen (g) | Weight after a full rotation of Pen (g) | Dispensed Material (g) | # of "Clicks" |
|---|---|---|---|---|
| 1 | 15.4 | 15.36 | 0.04 | 25 |
| 2 | 15.33 | 15.23 | 0.1 | 30 |
| 3 | 15.18 | 15.08 | 0.1 | 25 |
| 4 | 15.04 | 14.92 | 0.12 | 30 |
| 5 | 14.89 | 14.78 | 0.11 | 25 |

$$Average\ Dispensed\ Material\ per\ full\ rotation = \frac{\sum Dispensed\ Material}{\sum \#\ of\ Clicks} * 24$$

Formula I

The total dispensed material was divided by the total # of clicks shown in TABLE 2. This resulted in 0.0039 g per "click." As the # of "clicks" per full rotation was determined be 24, this number was multiplied by 24 to obtain the amount dispensed per full rotation of the rotating portion. The average dispensed material per full rotation was determined to be 0.094 g.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1.  A dispensing device (10) comprising:

    (a) an oral care composition, wherein the composition comprises fluoride, peroxide, a metal ion, or combinations thereof,
    (b) a body (12);
    (c) a rotating portion (13);
    (d) a dosing indicator, the dosing indicator comprising only:

        (i) a first dosing indicator symbol (14) on the body (12), and
        (ii) a second dosing indicator symbol (15) on the rotating portion (13),

    wherein a full rotation of the rotating portion (13) is indicated by the first dosing indicator symbol (14) and the second dosing indicator symbol (15) start aligned, and after the rotation of the rotating portion (13) in a single direction, the first dosing indicator symbol (14) and second dosing indicator symbol (15) end aligned, wherein a full rotation of the rotating portion (13) releases from about 0.05g to about 0.25g of the composition.

2.  The device (10) of claim 1, wherein the fluoride comprises stannous fluoride, sodium fluoride, amine fluoride, sodium monofluorophosphate, or combinations thereof.

3.  The device (10) of claim 1 or 2, wherein the metal ion comprises stannous, zinc, copper, or combinations thereof.

4.  The device (10) of any one of claim 1 to 3, wherein the peroxide comprises hydrogen peroxide, carbamide peroxide, or combinations thereof, preferably wherein the peroxide comprises hydrogen peroxide or carbamide peroxide.

5.  The device (10) of any one of claims 1 to 4, wherein the device (10) comprises an applicator (25), preferably wherein the device (10) also comprises a reservoir (20), the reservoir (20) comprising the oral care composition, more preferably wherein the applicator (25) comprises one or more orifices in fluid communication with the reservoir (20).

6.  The device (10) of any one of claims 1 to 5, wherein the first dosing indicator symbol (14) comprises an arrow, a line, a circle, an oval, a letter, a word, a picture, a graphic, or combinations thereof, preferably wherein the second indicator dosing symbol (15) comprises an arrow, a line, a circle, an oval, a letter, a word, a picture, a graphic, or combinations thereof, more preferably wherein the first and second dosing indicator symbols (14) are identical.

7.  The device (10) of claim 6, wherein the second dosing indicator symbol (15) comprises a volume scale to denote the volume of material dispensed per distance rotated.

8.  The device (10) of any one of claims 1 to 7, wherein the device (10) is free of bristles.

9.  The device (10) of any one of claims 1 to 8, wherein the device (10) is a whitening pen and the oral care composition is a whitening composition.

10. The device (10) of any one of claims 1 to 9, wherein the body (12) comprises a reservoir (20), the reservoir (20) comprising the whitening composition.

11. The device (10) of any one of claims 1 to 10, wherein the applicator (25) comprises one or more orifices in fluid communication with the reservoir (20).

12. The device (10) of any one of claims 6 to 11, wherein the first dosing indicator symbol (14) and the second indicator symbol (15) are portions of a graphic, figure, word, letter, number, symbol, or combinations thereof that, when aligned, display the entire graphic, figure, word, letter, number, symbol, or combinations thereof.

13. The device (10) of any one of claims 9 to 12, wherein about 0.1g of the whitening composition is dispensed with one full rotation of the rotation portion (13).


**Patentansprüche**

1. Abgabevorrichtung (10), umfassend:

   (a) eine Mundpflegezusammensetzung, wobei die Zusammensetzung Fluorid, Peroxid, ein Metallion oder Kombinationen davon umfasst;
   (b) einen Körper (12);
   (c) einen sich drehenden Abschnitt (13);
   (d) einen Dosierindikator, der Dosierindikator umfassend nur:

      (i) ein erstes Dosierindikatorsymbol (14) an dem Körper (12), und
      (ii) ein zweites Dosierindikatorsymbol (15) an dem rotierenden Abschnitt (13),

   wobei eine volle Drehung des sich drehenden Abschnitts (13) dadurch angezeigt wird, dass das erste Dosierindikatorsymbol (14) und das zweite Dosierindikatorsymbol (15) zueinander ausgerichtet beginnen, und nach der Drehung des sich drehenden Abschnitts (13) in eine einzelne Richtung, das erste Dosierindikatorsymbol (14) und das zweite Dosierindikatorsymbol (15) zueinander ausgerichtet enden, wobei eine volle Drehung des sich drehenden Abschnitts (13) von etwa 0,05 g bis etwa 0,25 g der Zusammensetzung freisetzt.

2. Vorrichtung (10) nach Anspruch 1, wobei das Fluorid Zinnfluorid, Natriumfluorid, Aminfluorid, Natriummonofluorphosphat oder Kombinationen davon umfasst.

3. Vorrichtung (10) nach Anspruch 1 oder 2, wobei das Metallion Zinn, Zink, Kupfer oder Kombinationen davon umfasst.

4. Vorrichtung (10) nach einem der Ansprüche 1 bis 3, wobei das Peroxid Wasserstoffperoxid, Carbamidperoxid oder Kombinationen davon umfasst, vorzugsweise wobei das Peroxid Wasserstoffperoxid oder Carbamidperoxid umfasst.

5. Vorrichtung (10) nach einem der Ansprüche 1 bis 4, wobei die Vorrichtung (10) einen Applikator (25) umfasst, vorzugsweise wobei die Vorrichtung (10) auch ein Reservoir (20) umfasst, das Reservoir (20) umfassend die Mundpflegezusammensetzung, mehr bevorzugt wobei der Applikator (25) eine oder mehrere Öffnungen umfasst, die in Fluidaustausch mit dem Reservoir (20) stehen.

6. Vorrichtung (10) nach einem der Ansprüche 1 bis 5, wobei das erste Dosierindikatorsymbol (14) einen Pfeil, eine Linie, einen Kreis, ein Oval, einen Buchstaben, ein Wort, ein Bild, eine Grafik oder Kombinationen davon umfasst, vorzugsweise wobei das zweite Indikatordosiersymbol (15) einen Pfeil, eine Linie, einen Kreis, ein Oval, einen Buchstaben, ein Wort, ein Bild, eine Grafik oder Kombinationen davon umfasst, mehr bevorzugt wobei das erste und das zweite Dosierindikatorsymbol (14) identisch sind.

7. Vorrichtung (10) nach Anspruch 6, wobei das zweite Dosierindikatorsymbol (15) eine Volumenskala umfasst, um das Volumen des pro gedrehten Abstand abgegebenen Materials zu bezeichnen.

8. Vorrichtung (10) nach einem der Ansprüche 1 bis 7, wobei die Vorrichtung (10) frei von Borsten ist.

**9.** Vorrichtung (10) nach einem der Ansprüche 1 bis 8, wobei die Vorrichtung (10) ein Aufhellungsstift ist und die Mundpflegezusammensetzung eine Aufhellungszusammensetzung ist.

**10.** Vorrichtung (10) nach einem der Ansprüche 1 bis 9, wobei der Körper (12) ein Reservoir (20) umfasst, das Reservoir (20) umfassend die Aufhellungszusammensetzung.

**11.** Vorrichtung (10) nach einem der Ansprüche 1 bis 10, wobei der Applikator (25) eine oder mehrere Öffnungen umfasst, die in Fluidaustausch mit dem Reservoir (20) stehen.

**12.** Vorrichtung (10) nach einem der Ansprüche 6 bis 11, wobei das erste Dosierindikatorsymbol (14) und das zweite Indikatorsymbol (15) Abschnitte einer Grafik, einer Figur, eines Wortes, eines Buchstabens, einer Zahl, eines Symbols oder Kombinationen davon sind, die, wenn sie zueinander ausgerichtet sind, die gesamte Grafik, die Figur, das Wort, den Buchstaben, die Zahl, das Symbol oder Kombinationen davon anzeigen.

**13.** Vorrichtung (10) nach einem der Ansprüche 9 bis 12, wobei etwa 0,1 g der Aufhellungszusammensetzung bei einer vollen Drehung des sich drehenden Abschnitts (13) abgegeben wird.


**Revendications**

**1.** Dispositif de distribution (10) comprenant :

(a) une composition d'hygiène buccale, dans lequel la composition comprend du fluorure, du peroxyde, un ion métallique, ou des combinaisons de ceux-ci ;
(b) un corps (12) ;
(c) une partie rotative (13) ;
(d) un indicateur de dosage, l'indicateur de dosage comprenant uniquement :

(i) un premier symbole indicateur de dosage (14) sur le corps (12), et
(ii) un deuxième symbole indicateur de dosage (15) sur la partie rotative (13),

dans lequel une rotation complète de la partie rotative (13) est indiquée par le premier symbole indicateur de dosage (14) et le second symbole indicateur de dosage (15) commence aligné, et après la rotation de la partie rotative (13) dans une seule direction, le premier symbole indicateur de dosage (14) et le second symbole indicateur de dosage (15) finissent alignés, dans lequel une rotation complète de la partie rotative (13) libère d'environ 0,05 g à environ 0,25 g de la composition.

**2.** Dispositif (10) selon la revendication 1, dans lequel le fluorure comprend du fluorure stanneux, du fluorure de sodium, du fluorure d'amine, du monofluorophosphate de sodium, ou des combinaisons de ceux-ci.

**3.** Dispositif (10) selon la revendication 1 ou 2, dans lequel l'ion métallique comprend un ion stanneux, de zinc, de cuivre, ou des combinaisons de ceux-ci.

**4.** Dispositif (10) selon l'une quelconque des revendications 1 à 3, dans lequel le peroxyde comprend du peroxyde d'hydrogène, du peroxyde de carbamide, ou des combinaisons de ceux-ci, de préférence dans lequel le peroxyde comprend du peroxyde d'hydrogène ou du peroxyde de carbamide.

**5.** Dispositif (10) selon l'une quelconque des revendications 1 à 4, dans lequel le dispositif (10) comprend un applicateur (25), de préférence dans lequel le dispositif (10) comprend également un réservoir (20), le réservoir (20) comprenant la composition d'hygiène buccale, plus préférablement dans lequel l'applicateur (25) comprend un ou plusieurs orifices en communication fluidique avec le réservoir (20).

**6.** Dispositif (10) selon l'une quelconque des revendications 1 à 5, dans lequel le premier symbole indicateur de dosage (14) comprend une flèche, une ligne, un cercle, un ovale, une lettre, un mot, une image, un graphique, ou des combinaisons de ceux-ci, de préférence dans lequel le second symbole de dosage d'indicateur (15) comprend une flèche, une ligne, un cercle, un ovale, une lettre, un mot, une image, un graphique, ou des combinaisons de ceux-ci, plus préférablement dans lequel les premier et second symboles indicateurs de dosage (14) sont identiques.

**7.** Dispositif (10) selon la revendication 6, dans lequel le second symbole indicateur de dosage (15) comprend une échelle volumique pour désigner le volume de matériau distribué par distance de rotation.

**8.** Dispositif (10) selon l'une quelconque des revendications 1 à 7, dans lequel le dispositif (10) est exempt de poils.

**9.** Dispositif (10) selon l'une quelconque des revendications 1 à 8, dans lequel le dispositif (10) est un stylo de blanchissement et la composition d'hygiène buccale est une composition de blanchissement.

**10.** Dispositif (10) selon l'une quelconque des revendications 1 à 9, dans lequel le corps (12) comprend un réservoir (20), le réservoir (20) comprenant la composition de blanchissement.

**11.** Dispositif (10) selon l'une quelconque des revendications 1 à 10, dans lequel l'applicateur (25) comprend un ou plusieurs orifices en communication fluidique avec le réservoir (20).

**12.** Le dispositif (10) selon l'une quelconque des revendications 6 à 11, dans lequel le premier symbole indicateur de dosage (14) et le second symbole indicateur (15) sont des parties de graphique, de figure, de mot, de lettre, de chiffre, de symbole, ou de combinaisons de ceux-ci qui, lorsqu'ils sont alignés, affichent l'ensemble graphique, figure, mot, lettre, chiffre, symbole, ou des combinaisons de ceux-ci.

**13.** Dispositif (10) selon l'une quelconque des revendications 9 à 12, dans lequel environ 0,1 g de la composition de blanchissement est distribué avec une rotation complète de la partie de rotation (13).

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20167067123 A
- WO 2005037127 A1
- US 20140011163 A1
- US 7201527 B
- US 8602774 B
- US 20060275225
- US 20180133119
- US 20180133121
- US 20080081023 A1

**Non-patent literature cited in the description**

- *CHEMICAL ABSTRACTS,* 134499-38-0